# EUROPEAN PATENT APPLICATION

(11) **EP 4 748 323 A1**
(43) Date of publication of application: **27.05.2026**
(21) Application number: 24842497.0
(22) Date of filing: 18.07.2024
(51) Int. Cl.: A61B 17/34, A61B 1/313

(54) **TROCAR FOR ELECTROMAGNETIC NAVIGATION WITH A VENTRICULOSCOPE**

(30) Priority: 19.07.2023 ES 202331334 U
(71) Applicant: Servicio Andaluz de Salud, 41071 Sevilla (ES)
(72) Inventor: GOMAR ALBA, Mario, 41071 Sevilla (ES); RUIZ GARCÍA, José Luis, 41071 Sevilla (ES); PLA RUIZ, José Miguel, 41071 Sevilla (ES); HUETE ALLUT, Antonio, 41071 Sevilla (ES); SAUCEDO, Leandro Ricardo, 41071 Sevilla (ES); GARCÍA PÉREZ, Fernando, 41071 Sevilla (ES); GUIL IBÁÑEZ, José Javier, 41071 Sevilla (ES)
(74) Representative: San Martin Alarcia, Esther
(86) International application number: PCT/ES2024/070460
(87) International publication number: WO 2025/017237

(57) **Abstract**

The present invention relates to a trocar for electromagnetic navigation with a ventriculoscope, comprising a base from which a rod protrudes, the base being shaped as a body of revolution configured to fit into the rear end of the tube of a sheath of a ventriculoscope, and the rod being cylindrical in shape with a rounded distal end for insertion through the tube of the sheath of a ventriculoscope. The invention is characterised in that it further comprises an open blind longitudinal hole at the proximal end of the base, said blind longitudinal hole being configured to receive a stylet of an electromagnetic navigation system.

## Description

### SUBJECT OF THE INVENTION

The present invention belongs to the field of neurosurgery, and more particularly to instruments used to access a patient's ventricular cavity.

The object of the present invention is a new trocar specifically designed to allow electromagnetic navigation of a ventriculoscope during the procedure of accessing a patient's brain ventricle.

### BACKGROUND OF THE INVENTION

There are four anatomical cavities in the brain, called cerebral ventricles, which are interconnected and constitute the ventricular system through which the cerebrospinal fluid circulates. Certain conditions, such as hydrocephalus, require access to the cerebral ventricles. For this, it is known a surgical instrument called "ventriculoscope".

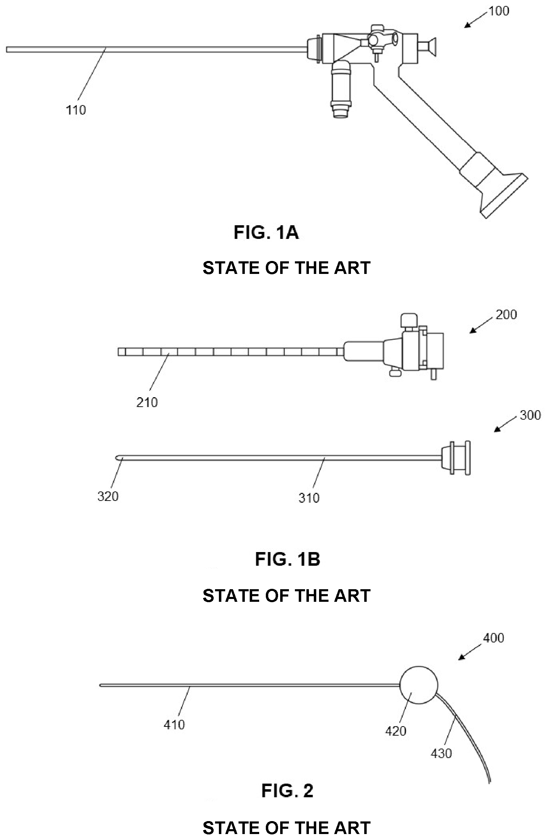

A ventriculoscope is a neurosurgical instrument designed to perform procedures in the ventricular cavities of the brain. FIG. 1A shows a ventriculoscope, specifically in this case, the LOTTA ventriculoscope model from Karl Storz. As can be seen, the ventriculoscope (100) has a main tube (110) along which several conduits (not visible in the figure) extend, which are used for vision, irrigation/suction, as well as for the introduction of specific instruments for each procedure. However, as the tube (110) of the ventriculoscope (100) is traversed by the aforementioned conduits, it has a tip with relatively sharp edges that is not suitable for moving through brain tissue to reach the ventricular space.

For this reason, in order to ensure that no damage is inflicted on the patient's brain tissue during the process of inserting the ventriculoscope into the ventricular cavity, it is known the use of two auxiliary elements designed specifically for this purpose: the sheath and the trocar.

FIG. 1B shows respective examples of a sheath (200, above) and a trocar (300, below) that are used in combination with the ventriculoscope (100) mentioned above. The sheath (200) essentially comprises a hollow tube (210) of a diameter greater than the diameter of the tube (110) of the ventriculoscope (100). As with the tube (110) of the ventriculoscope (100), the edges of the distal end of the tube (210) of the sheath (200) are also unsuitable for insertion through brain tissue, as they could cause tissue damage. The trocar (300), in turn, comprises a solid rod (310) that has a diameter smaller than the diameter of the tube (210) of the sheath (200) and a length slightly greater than the length of the tube (210) of the sheath (200). In addition, the tube (310) of the trocar (300) has an anatomically shaped distal end (320) formed by smooth curves. When the trocar (300) is inserted into the sheath (200), the tip of the tube (310) of the trocar (300) protrudes from the distal end of the tube (210) of the sheath (200).

The sheath (200) and trocar (300) are used in combination to facilitate insertion of the tube (110) of the ventriculoscope (100) into the ventricular cavity. First, the trocar (300) is inserted into the sheath (200) until the anatomical distal end (320) of the tube (310) of said trocar (300) protrudes from the end of the sheath (200). The sheath (200) is then inserted into the patient's brain cavity until reaching the ventricle using a suitable navigation system. Because the distal end (320) of the trocar is anatomical, tissue damage along the path is minimised. Once the sheath-trocar assembly has reached the ventricular space, the trocar (300) is removed and the tube (110) of the ventriculoscope (100) is inserted in its place. This procedure allows the ventriculoscope (100) to be inserted into the surgical field in an atraumatic manner.

Until now, the navigation systems used for this purpose were based on various techniques, among which optical navigation or insertion based on real-time echographic images. Sometimes the ventriculoscope tube is inserted directly, even though the edges of the distal end of the tube are not suitable for this purpose.

However, these navigation techniques have several drawbacks, being particularly desirable to be able to use electromagnetic navigation with this type of ventriculoscope.

### DESCRIPTION OF THE INVENTION

The inventors of the present application have developed a modified trocar that allows an electromagnetic navigation system to be used during the insertion of the ventriculoscope. In particular, the trocar is designed for use in combination with the LOTTA ventriculoscope model from Karl Storz.

Electromagnetic navigation is advantageous relative to other navigation systems, such as optical navigation, for various reasons. For example, it may be mentioned that the reference structures that must be attached to the base of the navigated element make it difficult to handle. In any case, the main concept underlying electromagnetic navigation is the introduction of a known element called stylet which, through certain electromagnetic means, is visible in a medical image by means of a system designed for this purpose.

FIG. 2 shows, by way of example, Medtronic's S8 Stealthsystem stylet. The stylet (400) essentially comprises a metallic needle (410) protruding from a flat circular body (420) which, in turn, is connected by a cable (430) to certain electronic means. The electromagnetic navigation system is designed so that the needle (410) is visible in real time through a medical imaging system.

Currently, the trocar used to introduce the ventriculoscope into the patient's ventricular cavity is not compatible with the use of the stylet of an electromagnetic navigation system.

The present invention solves that problem through a modification of the trocar used for the introduction of the ventriculoscope. This modification consists of making a longitudinal hole open at the rear end of the trocar and reaching a certain point along its length but which, in any case, is not open at its distal end. This hole is sized for the insertion of the EM stylet, so that the introduction of the sheath - with the trocar housed inside it as described above - is carried out with the aid of the magnetic navigation system based on the EM stylet.

Thus, the object of the present invention is a trocar for electromagnetic navigation with a ventriculoscope. The trocar comprises a base from which a rod protrudes. The base is shaped like a body of revolution configured to fit into the rear end of the tube of a ventriculoscope sheath (preferably the LOTTA ventriculoscope model from Karl Storz). The rod is cylindrically shaped with a rounded distal end for insertion through the tube of a ventriculoscope sheath (preferably the LOTTA model ventriculoscope from Karl Storz). The dimensions of the trocar of the invention are preferably 180 mm in length and 6 mm in diameter.

So far, we have essentially described the configuration of a trocar similar to that conventionally used to facilitate the insertion of a ventriculoscope (preferably **LOTTA** ventriculoscope model from Karl Storz). However, the trocar of the present invention differs from that in that it further comprises a blind longitudinal hole open at the proximal end of the base. This blind longitudinal hole is configured to receive a stylet of an electromagnetic navigation system (preferably a Medtronic S8 Stealthsystem stylet).

Thanks to this configuration, it is possible to insert an EM stylet into the longitudinal hole of the trocar, thus facilitating its electromagnetic navigation during the insertion process of the sheath-trocar assembly. In this way, it is possible to combine the advantages of using the sheath-trocar assembly for the insertion process with electromagnetic navigation.

The diameter of the blind longitudinal hole of the trocar will be adapted to the diameter of the stylet to be housed inside it. Preferably, the diameter of this hole is between 1.8 mm and 2.2 mm, even more preferably 2 mm.

As to the length of the blind longitudinal hole in the trocar, it will also be adapted to the length of the stylet to be inserted therein. Preferably, the length of said hole is between 150 mm and 170 mm, even more preferably 160 mm.

In principle, the trocar of the invention can be made of different materials, such as stainless steel or similar. However, in a particularly preferred embodiment of the invention, the trocar is manufactured using 3D printing. In that case, the material from which it is made is polypropylene.

### BRIEF DESCRIPTION OF THE DRAWINGS

FIG. 1A: shows an example of a ventriculoscope according to the prior art, specifically a LOTTA model ventriculoscope from Karl Storz.
FIG. 1B: shows a sheath (above) and a trocar (below) employed for the introduction of the ventriculoscope tube of FIG. 1A.
FIG. 2: shows a stylet of an electromagnetic navigation system according to the prior art, specifically the Medtronic S8 Stealthsystem stylet.

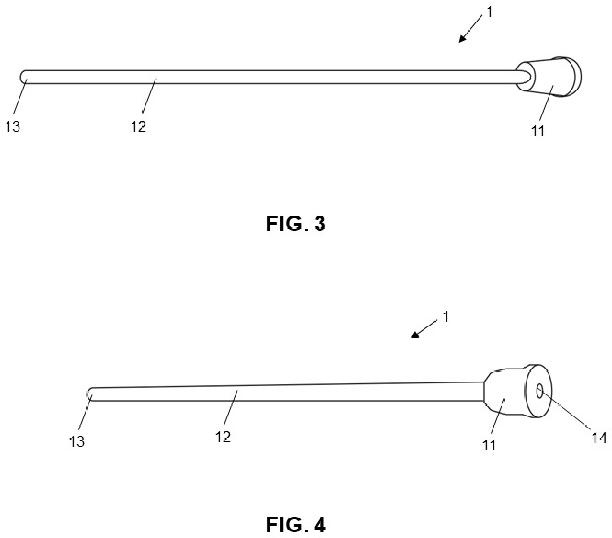

FIG. 3 shows a perspective view of the modified trocar according to the present invention.
FIG. 4 shows another perspective view of the modified trocar according to the present invention.

### PREFERRED EMBODIMENT OF THE INVENTION

A particular example of trocar (1) according to the present invention is described below with reference to the figures.

The trocar (1) comprises a base (11) and a rod (12). The base (11) is a body of revolution configured to fit into the rear portion of the sheath (200) of a ventriculoscope (100) (specifically, the LOTTA ventriculoscope model from Karl Storz). The rod (12) is a cylindrical body that fits inside the sheath (200) of the ventriculoscope (100). It is a cylindrical body of 6 mm in diameter and 180 mm in length provided with a rounded distal end (13). The base (11), in turn, has an approximate length of 15 mm.

The rod (12) is traversed by a blind longitudinal hole (14) of 2 mm in diameter. This hole (14) is open at the proximal end of the base (11), and its distal end is closed inside the rod (12). The hole (14) has a length of 16 cm (length inside the rod (12)), so that the distance to the distal end (13) of the trocar (1) is approximately 2 cm.

Thus, during the introduction of the sheath (200)-trocar (1) assembly, the rounded distal end (13) of the trocar (1) protrudes through the end of the sheath (200), thus providing the assembly with an anatomically shaped distal end that facilitates introduction. Furthermore, by inserting an EM stylet (400) along the hole (14), it is possible to visualise the position of the trocar (1) in real time using the electromagnetic navigation system.

## Claims

1. Trocar (1) for electromagnetic navigation with ventriculoscope, comprising a base (11) from which a rod (12) protrudes, wherein the base (11) is shaped as a body of revolution configured to fit into the rear end of the tube (210) of a sheath (200) of a ventriculoscope (100), and wherein the rod (12) has a cylindrical shape with a rounded distal end (13) for its insertion through the tube (210) of the sheath (200) of a ventriculoscope, **characterised in that** it further comprises a blind longitudinal hole (14) open at the proximal end of the base (11), said blind longitudinal hole (14) being configured to receive a stylet (400) of an electromagnetic navigation system.

2. Trocar (1) according to claim 1, wherein the blind longitudinal hole (14) has a diameter of between 1.8 mm and 2.2 mm.

3. Trocar (1) according to claim 2, wherein the blind longitudinal hole (14) has a diameter of 2.0 mm.

4. Trocar (1) according to any of the preceding claims, wherein the blind longitudinal hole (14) has a length of between 150 mm and 170 mm.

5. Trocar (1) according to claim 4, wherein the blind longitudinal hole (14) has a length of 160 mm.

6. Trocar (1) according to any of the preceding claims, which is manufactured by 3D printing.
